Europäisches Patentamt

European Patent Office

Office européen des brevets

(19)

(11) Veröffentlichungsnummer : **0 421 237 B1**

(12) **EUROPÄISCHE PATENTSCHRIFT**

(45) Veröffentlichungstag der Patentschrift :
**27.10.93 Patentblatt 93/43**

(21) Anmeldenummer : **90118367.3**

(22) Anmeldetag : **25.09.90**

(51) Int. Cl.⁵ : **C07C 255/16,** C07C 253/34

(54) **Verfahren zur Reinigung von Roh-Cyanhydrinen mit 3 bis 6 Kohlenstoffatomen.**

(30) Priorität : **05.10.89 DE 3933207**

(43) Veröffentlichungstag der Anmeldung :
**10.04.91 Patentblatt 91/15**

(45) Bekanntmachung des Hinweises auf die
Patenterteilung :
**27.10.93 Patentblatt 93/43**

(84) Benannte Vertragsstaaten :
**AT BE DE ES FR GB IT**

(56) Entgegenhaltungen :
**EP-A- 0 184 694**
**DE-A- 1 944 754**

(56) Entgegenhaltungen :
**US-A- 3 742 016**
**"Ullmann's Encyclopedia of Industrial Chemi-**
**stry", Ausgabe 5, Band A1: "Abrasives to**
**aluminum oxide", Seiten 91,91, VCH**

(73) Patentinhaber : **Degussa Aktiengesellschaft**
**Weissfrauenstrasse 9**
**D-60311 Frankfurt (DE)**

(72) Erfinder : **Buder, Wolfgang, Dr.**
**Hemkunt Chambers, 3rd Floor, 89, Nehru**
**Place**
**New Delhi - 110 019 (IN)**
Erfinder : **Rudolph, Udo**
**Grünaustrasse 11**
**D-6450 Hanau 9 (DE)**

## Beschreibung

Die Erfindung betrifft ein Verfahren zur Reinigung von Roh-Cyanhydrinen mit 3 bis 6 C-Atomen, wobei die Roh-Cyanhydrine aus Cyanwasserstoff und der entsprechenden niedrigsiedenden Carbonylverbindung gebildet worden sind und die genannten Reaktanden als Verunreinigungen enthalten. Die nicht umgesetzten Reaktanden werden destillativ unter Vakuum von den Roh-Cyanhydrinen abgetrennt und der Gewinnung von Cyanhydrinen zugeführt. Das Verfahren richtet sich insbesondere auf die Reinigung von Acetoncyanhydrin.

Cyanhydrine lassen sich in bekannter Weise durch basekatalysierte Anlagerung von Cyanwasserstoff an Carbonylverbindungen, wie Aldehyde und Ketone, gewinnen. Cyanhydrine mit 3 bis 6 C-Atomen stellen technisch wertvolle Zwischenprodukte dar. Besondere Bedeutung kommt dem Acetoncyanhydrin zu, das in großer Menge zu Methacrylsäureestern, aber auch zu Dimethylhydantoinderivaten und Azobisisobutyronitril weiterverarbeitet wird.

Zur Herstellung von Folgeprodukten von Cyanhydrinen müssen diese oft in sehr reiner Form, im allgemeinen über 97 %ig, zur Verfügung stehen. Wesentliche Nebenbestandteile in rohen Cyanhydrinen sind nicht umgesetzte Reaktanden, also Cyanwasserstoff und die niedrigsiedende Carbonylverbindung, da die Bildung und Dissoziation von Cyanhydrinen ein Gleichgewicht bilden. Während die Cyanhydrinbildung basisch katalysiert wird, werden die rohen Cyanhydrine vor der Aufarbeitung bzw. Reinigung durch Zugabe einer Säure stabilisiert.

Es ist bekannt, Acetoncyanhydrin durch kontinuierliche Einspeisung der Reaktanden Aceton und Cyanwasserstoff in alkalisch katalysierte Reaktionskreisläufe oder in zu Strömungsrohren ausgebildeten Reaktoren herzustellen, wobei die bei der jeweiligen Reaktionstemperatur - im allgemeinen im Bereich von 0-50 °C - aus dem Dissoziationsgleichgewicht resultierende Konzentration an Acetoncyanhydrin im Reaktionsgemisch gebildet wird. Um einen hohen Umsatz in praktikabler Zeit zu erzielen, erfolgt die Umsetzung meist in mehreren Stufen bei abnehmender Temperatur. Anschließend wird der Katalysator neutralisiert und, soweit erforderlich, abgetrennt und das Roh-Acetoncyanhydrin schwach sauer stabilisiert, so daß die nicht abreagierten Ausgangsstoffe unter Vakuum bei Temperaturen bis 80 °C abgezogen werden können. Soweit dies möglich ist, werden die abdestillierten Reaktanden dem Cyanhydrinreaktor wieder zugeführt - vgl. Ullmann's Encyclopedia of Industrial Chemistry, 5th ed. (1985), Seite 91-92.

Zur Vakuumerzeugung bei der Reinigung des Roh-Cyanhydrins werden in den bisher bekannten Verfahren übliche Apparate, wie Flüssigkeitsringpumpen, Dampfstrahler oder Wälzkolbengebläse eingesetzt. Diese Systeme haben jedoch entscheidende Nachteile: Um das erforderliche Vakuum zu erzeugen, bedarf es mehrstufiger und damit aufwendiger und teilweise sehr reparaturanfälliger Systeme. Solchen Systemen müssen ferner komplizierte Anlagen zur Abwasseraufarbeitung und/oder Abgaswäsche nachgeschaltet werden. Es kommt hinzu, daß die abgezogenen Reaktanden vor der Rückführung in den Cyanhydrinreaktor zwischenkondensiert werden mußten.

Die Aufgabe der Erfindung besteht somit darin, die Abtrennung der nicht umgesetzten Reaktanden der Cyanhydrinsynthese aus Cyanwasserstoff und einer Carbonylverbindung - Aldehyden und Ketonen mit 2 bis 5 C-Atomen - zu vereinfachen, um damit das Cyanhydrin zu reinigen. Die Reinigungsstufe sollte mit solchen Betriebssystemen erfolgen, welche bei möglichst niedriger Investitionssumme kaum störanfällig sind, eine praktisch quantitative Rückführung der Reaktanden erlauben und in einfacher Weise in den Reaktionskreislauf eingebunden werden können.

Diese Aufgabe wird gelöst durch ein Verfahren zur Reinigung von Roh-Cyanhydrinen mit 3 bis 6 Kohlenstoffatomen, welche Cyanwasserstoff und die ihnen zugrundeliegende Carbonylverbindung aus der Cyanhydrinherstellung enthalten, durch Abdestillieren der nicht umgesetzten Reaktanden unter Vakuum und Gewinnung des Cyanhydrins aus den abgetrennten Reaktanden, das dadurch gekennzeichnet ist, daß man das Vakuum mit einer Flüssigkeitsstrahlpumpe erzeugt, die bei der Destillation entstehenden Brüden, welche Cyanhydrin-Reaktanden enthalten, ohne oder nach teilweiser Kondensation in den Treibstrahl der Pumpe leitet, die Flüssigkeitsstrahlpumpe mit basischen Katalysator enthaltendem Roh-Cyanhydrin betreibt und die Flüssigkeitsstrahlpumpe in einen gekühlten Reaktionskreislauf eingebunden ist, in dem die aus den Brüden absorbierten Cyanhydrin-Reaktanden zum Cyanhydrin abreagieren.

Die Unteransprüche richten sich auf bevorzugte Ausführungsformen des erfindungsgemäßen Verfahrens zur Reinigung von Roh-Aceton-Cyanhydrin.

Es war nicht vorhersehbar, daß die vorgenannten Aufgaben durch den Einsatz einer mit Roh-Cyanhydrin betriebenen Flüssigkeitsstrahlpumpe, welche in einen einfachen Reaktionskreislauf aus im wesentlichen der Flüssigkeitsstrahlpumpe, einem Reaktor und einer Pumpe und ggf. einem Wärmeaustauscher eingebunden ist, in einfacher Weise gelöst werden konnten. Durch die Verwendung einer Flüssigkeitsstrahlpumpe ist es nun möglich, ohne die Verwendung zusätzlicher Vakuumpumpen und/oder Kondensatoren, das erforderliche Vakuum im Bereich von im allgemeinen 30-100 mbar zu erzeugen und die aus dem Roh-Cyanhydrin abdestillierten Reaktanden direkt in einem Reaktionskreislauf zu absorbieren und zu Cyanhydrin abreagieren zu lassen. Die Flüssigkeitsstrahlpumpe kann mit einer üblichen Düse ausgestattet sein. Um im Falle der Abwesenheit von Inertgasen Kavitation in der Düse zu vermeiden und/oder die Gas-Flüssigkeits-Austauschfläche zu vergrößern, kann die Düse auch mehrfach geschlitzt sein - für Flüssigkeitsstrahlpumpen mit solchen oder ähnlich ausgebildeten Düsen ist auch der Begriff Flüssigkeitsstrahlkondensatoren gebräuchlich.

Das erfindungsgemäße Verfahren kann zur Reinigung von rohen Cyanhydrinen mit 3 bis 6 C-Atomen, vorzugsweise zur Reinigung von Roh-Cyanhydrinen mit einem Gehalt von über 90 Gew.-% neben 1-5 Gew.-% Cyanwasserstoff und 1-7 Gew.-% Carbonylverbindung, herangezogen werden. Besonders geeignet ist das Verfahren zur Reinigung von rohem Lactonitril, 2-Hydroxy-n-butyronitril, Aceton- und Methyl-ethylketoncyanhydrin; ganz besonders bevorzugt wird rohes Acetoncyanhydrin gereinigt.

Die bei der Destillation des Roh-Cyanhydrins entstehenden Brüden enthalten außer Cyanwasserstoff und der Carbonylverbindung im allgemeinen etwas Wasser und etwas Cyanhydrin. Das Mengenverhältnis Cyanwasserstoff zu Carbonylverbindung in den Brüden hängt sowohl von den Druck- und Temperaturbedingungen des eingesetzten Verdampfers als auch dem in der Cyanhydrinsynthese eingestellten stöchiometrischen Verhältnis der Reaktanden ab. Im Falle des Einsatzes einer höhersiedenden Carbonylverbindung und/oder eines HCN-Überschusses werden die Brüden einen ensprechend hohen Anteil an HCN enthalten.

Erfindungsgemäß wird die Flüssigkeitsstrahlpumpe mit rohem Cyanhydrin betrieben, das eine ausreichende Menge basischen Katalysator enthält oder über einen basischen Festbettkatalysator umgewälzt wird, so daß die im Roh-Cyanhydrin absorbierten Brüdenbestandteile zum entsprechenden Cyanhydrin umgesetzt werden. Dem Reaktionskreislauf, in welchen die Flüssigkeitsstrahlpumpe eingebunden ist, werden die an sich bekannten Katalysatoren, wie Alkalihydroxide, Alkalicarbonate, Amine oder Anionenaustauscher zugesetzt. Soweit erforderlich, kann in den genannten Reaktionskreislauf zusätzlich Carbonylverbindung eingespeist werden, um das gewünschte Molverhältnis der Reaktanden einzustellen. Dies kann insbesondere dann erforderlich werden, wenn die Brüden zu wenig Carbonylverbindung enthalten.

Der genannte Reaktionskreislauf kann in den Cyanhydrin-Herstellungsprozeß integriert werden oder diesem nachgeschaltet sein. Die Reaktionstemperatur wird zwischen -10 °C und +30 °C, vorzugsweise zwischen -10 °C und +10 °C, gehalten. Da die Cyanhydrinbildung exotherm verläuft, das Gleichgewicht zwischen den Reaktanden und dem Cyanhydrin bei niedrigen Temperaturen zugunsten des Cyanhydrins verschoben ist und auch die Absorption der Brüden bei niedriger Temperatur bevorzugt ist und zu einem niedrigeren Dampfdruck des Systems führt, wird das im Reaktionskreislauf befindliche Reaktionsgemisch zweckmäßigerweise vor Eintritt desselben in die Flüssigkeitsstrahlpumpe gekühlt.

Aus dem nach einer ein- oder mehrstufigen Umsetzung, vorzugsweise bei abnehmender Temperatur, erhaltenen Reaktionsgemisch - Roh-Cyanhydrin genannt - werden nach Neutralisation des Katalysators und saurer Stabilisierung die nicht umgesetzten Reaktanden abdestilliert, worunter auch eine Desorption verstanden wird. Prinzipiell können für diese Destillation bzw. Desorption Verdampfer unterschiedlicher Bauart eingesetzt werden. Wegen der thermischen Labilität der Cyanhydrine werden solche Systeme bevorzugt, welche nur zu einer mäßigen und insbesondere kurzfristigen thermischen Belastung führen; Dünnschichtverdampfer, wie Fallfilmverdampfer werden bevorzugt. Die Abtrennung der Reaktanden vom Roh-Cyanhydrin erfolgt vorzugsweise im Bereich von 30-90 °C, bei einem Druck von 35-100 mbar. Eine Temperatur über 90 °C ist im allgemeinen weniger empfehlenswert; bei einer Temperatur um/unter 30 °C läßt sich die Carbonylverbindung, ausgenommen ggf. Acetaldehyd, nicht mehr befriedigend aus dem Reaktionsgemisch abdestillieren/desorbieren. Bei der Reinigung von Roh-Acetoncyanhydrin wird der Verdampfer vorzugsweise bei 40-90 °C und einem Druck von 35-100 mbar, insbesondere 40-60 mbar betrieben. Die Brüden des Verdampfers können mittels der Flüssigkeitsstrahlpumpe direkt vom Roh-Cyanhydrin absorbiert werden; sofern erwünscht, kann zur teilweisen Kondensation der Brüden zur Entlastung der Flüssigkeitsstrahlpumpe ein einfacher Kondensator zwischengeschaltet werden, wobei auch das Kondensat dem Reaktionskreislauf zugeführt wird.

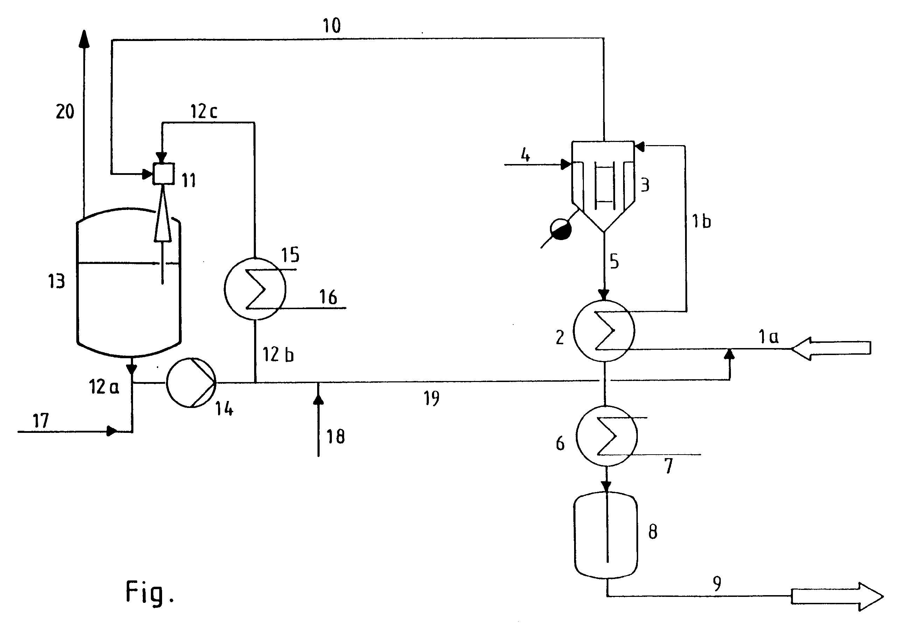

Eine bevorzugte Ausführungsform des Verfahrens, wie sie auch dem Beispiel zugrundeliegt, ist der Figur zu entnehmen:

Durch Leitung (1a) wird das Roh-Cyanhydrin in stabilisierter Form - meist mit $H_2SO_4$ auf pH 1-3 eingestellt - eingespeist, Rein-Cyanhydrin wird durch Leitung (9) entnommen. Nach Passage eines Wärmeaustauschers (2) wird das erwärmte Roh-Cyanhydrin über Leitung (1b) dem Verdampfer (3), der über (4) beheizt wird, zugeführt. Die Brüden gelangen über Leitung (10) zur Flüssigkeitsstrahlpumpe (11), das gereinigte Cyanhydrin über Leitung (5) und die Wärmeaustauscher (2) und (6), wobei (6) durch (7) mit Kühlwasser gekühlt wird, in den Behälter (8). Durch die Flüssigkeitsstrahlpumpe (11) werden die Brüden (10) abgesaugt; letztere lösen sich spontan in der Betriebsflüssigkeit und reagieren im Reaktionskreislauf zu Cyanhydrin. Der Reaktionskreislauf, in welchen die Flüssigkeitsstrahlpumpe (11) eingebunden ist und in welchem sich Katalysator enthaltendes Roh-Cyanhydrin befindet, umfaßt ferner den Reaktor (13), die Pumpe (14), den Wärmeaustauscher (15) und die Umlaufleitung (12a), (12b) und (12c). Die Pumpe (14) sorgt für den erforderlichen Umlauf und Druck der Betriebsflüssigkeit vor der Flüssigkeitsstrahlpumpe (11); der durch Leitung (16) mit Kühlwasser oder Kühlsole betriebene Wärmeaustauscher (15) sorgt für die Kühlung der Betriebsflüssigkeit. Durch Leitung (17) können dem System die Carbonylverbindung und basischer Katalysator zugeführt werden. Um das Niveau im Reaktor (13) konstant zu halten, wird neu gebildetes Cyanhydrin kontinuierlich über Leitung (19) ausgeschleust, durch Zugabe einer Säure durch Leitung (18) neutralisiert und stabilisiert und dem durch Leitung (1a) fließenden stabilisierten Roh-Cyanhydrin zugegeben. Leitung (20) bedeutet die Abgasleitung, durch welche Inertgase mit im wesentlichen sehr geringem Gehalt an Cyanwasserstoff - entsprechend dem Partialdruck über der Flüssigkeit im Reaktor (13) - aus dem System abgeführt und einer Abgaswäsche, einer Entgiftung oder Verbrennung zugeleitet werden. Vorteilhaft ist es, die ggf. durch Leitung (17) zugespeiste Menge Carbonylverbindung zuvor zur Abgaswäsche zu verwenden. Die Kinetik der jeweiligen Cyanhydrinbildung bei der im Reaktor (13) herrschenden Temperatur bestimmt die nötige Verweilzeit und damit das Volumen des Reaktors.

Das erfindungsgemäße Verfahren ist, wie gezeigt wurde, einfach handhabbar und läßt sich unter Verwendung einfacher Apparate sicher betreiben. Erfindungsgemäß kann der Gehalt an Cyanhydrin von 90-95 Gew.-% problemlos auf über 97 Gew.-% gesteigert werden.

Beispiel

Das nachfolgende Beispiel richtet sich auf die Reinigung von Roh-Acetoncyanhydrin (=Roh-ACH) in einer Pilotanlage gemäß Figur.

Betriebsbedingungen

| | |
|---|---|
| Tempertur des in den Verdamper (3) eingespeisten Roh-ACH | 40 °C |
| Temperatur des aus dem Verdampfer (3) ablaufenden Rein-ACH | 85 °C |
| Destillationsdruck im Verdampfer (3) | 50 mbar |
| Temperatur des Reaktionsgemisches im Reaktor (13) | 0 °C |
| Temperatur des Reaktionsgemisches in der Leitung (12c) vor der Flüssig-keitsstrahlpumpe (11) | -3 °C |
| Druck des Reaktionsgemisches in der Leitung (12c) vor der Flüssig-keitsstrahlpumpe (11) | 5 bar |
| Durch Leitung (1a/1b) in den Verdampfer (3) eingespeiste Menge an Roh-ACH | ca. 1500 kg/h ca. 94 gew.-%ig |
| Durch Leitung (9) abgezogene Menge Rein-ACH | ca. 1500 kg/h 98,5 gew.-%ig |
| Aus Reaktor (13) ausgeschleuste und via Leitung (19) zum Verdampfer rückgeführte Menge Roh-ACH | ca. 140 kg/h 90 gew.-%ig |

```
Via Leitung (17) zum Reaktions-
kreislauf dosierte Menge Aceton              ca. 25 kg/h

Brüdenmenge                                  ca. 115 kg/h

Zusammensetzung der Brüden
in Leitung (10)                              43 % ACH
                                             25 % HCN
                                             37 % Aceton

Durch Pumpe (14) umgewälzte Menge
des Absorptions-/Reaktions-
kreislaufes                                  ca. 20 m³/h
```

**Patentansprüche**

1. Verfahren zur Reinigung von Roh-Cyanhydrinen mit 3 bis 6 Kohlenstoffatomen, welche Cyanwasserstoff und die ihnen zugrundeliegende Carbonylverbindung aus der Cyanhydrinherstellung enthalten, durch Abdestillieren der nicht umgesetzten Reaktanden unter Vakuum und Gewinnung des Cyanhydrins aus den abgetrennten Reaktanden,
dadurch gekennzeichnet,
daß man das Vakuum mit einer Flüssigkeitsstrahlpumpe erzeugt, die bei der Destillation entstehenden Brüden, welche Cyanhydrin-Reaktanden enthalten, ohne oder nach teilweiser Kondensation in den Treibstrahl der Pumpe leitet, die Flüssigkeitsstrahlpumpe mit basischen Katalysator enthaltendem Roh-Cyanhydrin betreibt und die Flüssigkeitsstrahlpumpe in einen gekühlten Reaktionskreislauf eingebunden ist, in dem die aus den Brüden absorbierten Cyanhydrin-Reaktanden zum Cyanhydrin abreagieren.

2. Verfahren nach Anspruch 1,
dadurch gekennzeichnet,
daß man Roh-Acetoncyanhydrin, vorzugsweise ein solches mit einem Gehalt über 90 Gew.-% und 1-5 Gew.-% Cyanwasserstoff und 1-7 Gew.-% Aceton reinigt.

3. Verfahren nach Anspruch 2,
dadurch gekennzeichnet,
daß man die nicht umgesetzten Reaktanden bei 40-90 °C bei einem Druck von 35-100 mbar, vorzugsweise unter Verwendung eines Fallfilmverdampfers, vom Roh-Acetoncyanhydrin abdestilliert.

4. Verfahren nach den Ansprüchen 2 oder 3,
dadurch gekennzeichnet,
daß man die Temperatur im Reaktionskreislauf auf -10 °C bis +10 °C hält.

5. Verfahren nach einem oder mehreren der Ansprüche 2 bis 4,
dadurch gekennzeichnet,
daß man, soweit erforderlich, dem Reaktionskreislauf Aceton in einer solchen Menge zufügt, daß die Reaktanden in etwa stöchiometrischem Verhältnis anwesend sind.

**Claims**

1. Process for the purification of crude cyanhydrins with 3 to 6 carbon atoms, which contain hydrogen cyanide and the carbonyl compound on which they are based from the cyanhydrin production, by distilling off the unreacted reactants under vacuum and recovering the cyanhydrin from the separated reactants, characterized in that
the vacuum is generated with a liquid jet pump, the exhaust vapours arising during the distillation, which contain cyanhydrin reactants, are led without or after partial condensation into the driving jet of the pump, the liquid jet pump is driven with crude cyanhydrin containing basic catalyst and the liquid jet pump is integrated into a cooled reaction circuit in which the cyanhydrin reactants absorbed from the exhaust vapours react to completion to the cyanhydrin.

2. Process according to Claim 1,
characterized in that
crude acetone cyanhydrin, preferably with a concentration exceeding 90 % by weight and 1-5 % by weight of hydrogen cyanide and 1-7 % by weight of acetone, is purified.

3. Process according to Claim 2,
characterized in that
the unreacted reactants are distilled off from the crude acetone cyanhydrin at 40-90 °C and a pressure of 35-100 mbar, preferably using a falling film evaporator.

4. Process according to Claims 2 or 3,
characterized in that
the temperature in the reaction circuit is kept at -10 °C to +10 °C.

5. Process according to one or more of Claims 2 to 4,
characterized in that
as required, acetone is added to the reaction circuit in such an amount that the reactants are present in approximately stoichiometric ratio.

**Revendications**

1. Procédé de purification de cyanhydrines brutes ayant de 3 à 6 atomes de carbone, qui renferment de l'acide cyanhydrique et le composé carbonylé qui est à la base de celles-ci, à partir de la production de la cyanhydrine, par séparation par distillation des réactifs qui n'ont pas réagi sous vide et récupération de la cyanhydrine à partir des réactifs séparés, caractérisé en ce que l'on produit le vide avec une pompe à jet de liquide, on conduit les vapeurs qui se forment au cours de la distillation renfermant les réactifs de la cyanhydrine, sans ou après condensation partielle, dans le jet propulseur de la pompe, on actionne la pompe à jet de liquide avec la cyanhydrine brute contenant le catalyseur basique et la pompe à jet de liquide est incorporée dans un cycle de réaction refroidi, dans lequel les réactifs de cyanhydrine absorbés à partir des vapeurs, sont mis à réagir en cyanhydrine.

2. Procédé selon la revendication 1, caractérisé en ce que l'on purifie l'acétone cyanhydrine brute - de préférence celle ayant une teneur supérieure à 90 % en poids et de 1 à 5 % en poids d'acide cyanhydrique et de 1 à 7 % en poids d'acétone.

3. Procédé selon la revendication 2, caractérisé en ce que l'on sépare par distillation de l'acétonecyanhydrine, les réactifs qui n'ont pas réagi, à 40 - 90°C à une pression de 35-100 mbarsi, de préférence en utilisant un évaporateur à film tombant.

4. Procédé selon les revendications 2 ou 3, caractérisé en ce que l'on maintient la température dans le cycle de réaction de -10°C à +10°C.

5. Procédé selon l'une ou plusieurs des revendications 2 à 4, caractérisé en ce que l'on ajoute - pour autant que cela soit nécessaire - au cycle de réaction de l'acétone en quantité telle, que les réactifs soient présents en rapport à peu près stoechiométrique.

Fig.